# EUROPEAN PATENT SPECIFICATION

(11) **EP 4 400 073 B1**
(45) Date of publication and mention of the grant of the patent: **22.10.2025**
(21) Application number: 23150993.6
(22) Date of filing: 10.01.2023
(51) Int. Cl.: A61C 8/00, A61C 9/00

(54) **DENTAL SCAN AID, DENTAL SCAN AID SYSTEM AND DENTAL SCAN AID APPARATUS**
DENTALE SCAN-HILFE, DENTALES SCAN-HILFSSYSTEM UND DENTALES SCAN-HILFSGERÄT
AIDE AU BALAYAGE DENTAIRE, SYSTÈME D'AIDE AU BALAYAGE DENTAIRE ET APPAREIL D'AIDE AU BALAYAGE DENTAIRE

(43) Date of publication of application: 17.07.2024
(73) Proprietor: Nulty, Adam Brian, Bury, Lancashire BL9 0NT (GB)
(72) Inventor: Nulty, Adam Brian, Bury, Lancashire BL9 0NT (GB)
(74) Representative: Doherty, William

(56) References cited:
- WO-A1-2014/078412
- WO-A1-2021/087549
- WO-A1-2022/094301
- JP-A- 2019 217 138

## Description

The present invention relates to a dental scan aid, preferably, but not necessarily exclusively for use in scanning a maxillary or mandibular arch. The invention further relates to a dental scan aid system comprising such a dental scan aid, and to a dental scan aid apparatus.

Prior to performing dental treatment, dental professionals often scan a patient's jaw. An intra oral scanning device is commonly used and the scan is processed to provide a model and map of the area of interest. The model and map then allow a dental professional to design and prepare for said dental treatment. This is particularly common in restoration cases, where crowns and/or implants and/or bridges are required. It is imperative that the placement of such oral structures is accurate in a patient's maxillary and/or mandibular arch. If the placement is incorrect, patient discomfort, infection, gum recession, nerve and tissue damage can result. In order to ensure the placement is accurate, an accurate scan of the area of interest, and, in particular, any teeth and/or gingiva, is required.

Often, the maxillary or mandibular arch being scanned, and subsequently mapped, is largely or completely edentulous. During a scanning process, many individual small area scans are collected. These scan images are then stitched together to provide an overall scan of the area. The featureless nature of an edentulous area causes a loss of accuracy during the scanning process, and the resultant map of the maxillary or mandibular arch is distorted and less accurate. This can be a problem for large restorations, in particular for full arch implant bridges, as extremely accurate position information is required to ensure a passive fit. The loss of scan accuracy is unpredictable and therefore there is often doubt over the accuracy of the maxillary or mandibular arch scan, and any oral structures present. Currently, to improve the scan accuracy, a tedious, lengthy process of checking, sectioning, and verifying each individual small area scan is required. This is inefficient and increases both treatment time and cost.

Examples of oral scan structures known in the art are disclosed in WO2022/094301A1, JP2019217138A, and WO2021/087549A1.

It is an object of the present invention to obviate or overcome the above-referenced difficulties.

The invention is defined in the appended claims.

According to a first aspect of the invention, there is provided a dental scan aid for enhancing a scan accuracy of a maxillary or mandibular arch during a dental scan procedure, the dental scan aid comprising: a scan substrate; an oral attachment extending from the substrate, the oral attachment being attachable to an oral structure fixed relative to the said maxillary or mandibular arch, wherein the oral attachment is configured to be attached to an oral structure comprising any one of: an intraoral scan body and/or a tooth and/or an implant and/or a dental abutment; and the scan substrate at least in part has a randomised irregular morphology defined by a plurality of irregularly shaped void regions, the randomised irregular morphology thereby providing positional information relative to the maxillary or mandibular arch to improve dental scan accuracy when the oral attachment is attached to the said oral structure during a dental scan procedure .

Loss of scan accuracy, as mentioned above, is detrimental to subsequent dental procedures reliant on scan data. The present invention provides a dental scan aid that improves dental scan accuracy. The provision of a scan substrate with an irregular morphology aids the mapping and stitching together of scan images taken by an oral scanning device. The irregular morphology may beneficially be randomised. This may be particularly beneficial when the stitching software is aware of the design of the irregular morphology and so the scan substrate can function as a reference object for the scan data. The stitching software will be able to determine the relative position of the dental scan aid in a scanned frame, when compared to other oral features. This may be of particular use for highly edentulous areas where scan accuracy declines due to the featureless nature of edentulous areas.

Preferably, the oral attachment may be resiliently flexible to allow for push-fit attachment. A resiliently flexible oral attachment provides for a quick and straightforward way of attaching the oral attachment to the oral structure. Therefore, the dental procedural time may be reduced, and in turn patient comfort increased.

Optionally, the oral attachment may comprise a screw platform to allow for dental scan aid to be screwed to an oral structure.

Screwing the dental scan aid to an oral structure may provide increased security, and stability of the dental scan aid placement. It may reduce the risk of the dental scan aid moving prematurely from it position relative to the oral structure. Therefore, a screw platform as part of the oral attachment may provide for a more accurate scan. This may be of particular use in areas that are likely to result in movement, or, for patients who may accidentally move a dental scan aid when it is in use. It may also be of use in hard to reach areas, where a guide, such as a screw platform may be of use.

The oral attachment being able to attach to multiple types of oral structure increases the versatility and universal nature of the dental scan aid. It enables the dental scan aid to be used in different circumstances and for scans for different prospective dental procedures. For example, it allows for the dental scan aid to be used to scan for the fitting and placement of full arch implant bridges, by attachment to an intraoral scan body.

Preferably, the scan substrate may have a perimetric oral attachment support to which the oral attachment is connected, wherein the scan substrate has a central portion confined by the perimetric oral attachment support, and the central portion has the said irregular morphology.

The perimetric oral attachment support provides a simple way for the oral attachment and central portion to connect to one another. The perimetric oral attachment support also confines the central portion which is where the irregular morphology is preferably located. The irregular morphology may not extend past the perimetric oral attachment support. This may increase patient comfort.

The scan substrate is substantially planar.

A planar scan substrate ensures that the dental scan aid fits comfortably in a user's mouth and prevents the irregular morphology making unwanted contact with the user. This may also ease the manufacturing process. Although it is envisaged that there may be protrusions on the irregular morphology that do extend beyond the perimetric oral attachment support.

Optionally, the dental scan aid may comprise a plurality of said oral attachments extending from the scan substrate.

Having more than one oral attachment allows for the scan substrate to attach to more than one oral structure. Thus, the dental scan aid may function as a bridge between oral structures. More than one oral attachment may also increase the universal nature of the dental scan aid.

In a preferred embodiment, at least one oral attachment of the plurality of oral attachments may be different to one other oral attachment of the plurality of oral attachments.

Different oral attachments allow for use with different oral structures. The provision of multiple oral attachments increases the utility and versatility of the dental scan aid. The dental scan aid may be more universal in nature as the same aid design may be used with a variety of oral structures. For example, it may allow a dental scan aid to connect to both an intraoral scan body and a dental abutment at the same time to form a bridge between the two. Alternatively, it may be used to bridge two oral structures of the same type, such as two intraoral scan bodies, but the oral structures may be of assorted sizes, and so different sized oral attachments may be utilised. It may be that one dental scan aid has at least one attachment suitable for attachment to each of the various oral structures, thus the same design of dental scan aid can be used regardless of the oral structure.

Preferably, the scan substrate may have a length of no greater than 80 mm.

The scan substrate having a length less than the average width and/or length of a maxillary or mandibular arch may increase patient comfort. It may also increase ease of use by the dental professional.

The scan substrate has void regions.

The void regions may allow for the oral scanning device to scan the area underneath the scan substrate. This may be beneficial when stitching together the individual area scans to produce the overall scan of the maxillary or mandibular arch. The void regions can beneficially enhance the irregular morphology of the scan substrate.

According to a second aspect of the invention, there is provided a dental scan aid system comprising: a plurality of dental scan aids in accordance with the first aspect of the invention; and wherein at least one of the plurality of dental scan aids has a different irregular morphology to at least one other dental scan aid of the plurality of dental scan aids.

Providing a plurality of different dental scan aids may be useful for different dental procedures. It may be preferential to have multiple scan aids attached to oral structures fixed relative to the said maxillary or mandibular arch. The dental scan aids having different irregular morphologies may allow for increased scan accuracy. The different irregular morphology may allow for the software that stitches together the scan images collected by the dental scan to differentiate between the two dental scan aids.

Optionally, at least one of the plurality of dental scan aids has an oral attachment different to at least one other dental scan aid.

Different oral attachments allow for use with different oral structures. The provision of different oral attachments increases the versatility of the dental scan aid and allows dental scan aids to be used in different circumstances.

Preferably, at least one of the plurality of dental scan aids has a length and/or width different to at least one other dental scan aid.

In some cases, it may be preferential to use multiple small dental scan aids rather than one larger dental scan aid. Therefore, the provision of different sized dental scan aids is useful. Different sized dental scan aids may also be useful for different maxillary or mandibular arch sizes and different dental procedures, thereby providing a surgeon with a set of tools which can be utilised irrespective of the patient.

According to a third aspect of the invention, there is provided a dental scan aid apparatus comprising: a dental scan aid in accordance with the first aspect of the invention; intra oral scanner; and a processor for processing scan data collected by said intra oral scanner.

Providing a dental scan aid apparatus allows for a dental professional to have all of the necessary components to hand. It may also ensure compatibility between the dental scan aid and the intraoral scanner and processor.

In the state of the art, intraoral scanners are used to map a patient's maxillary or mandibular arch prior to dental procedures, to allow a dental professional to plan the procedure. Processing software is then used to stitch together multiple small scans performed by the intraoral scanner. Often the areas being scanned are edentulous, and thus largely featureless. The stitching together of such areas is troublesome, and results in a loss of scan accuracy. The present invention provides a solution to this problem. The present invention provides a scan substrate having an at least in part irregular morphology. The irregular morphology provides positional information relative to the maxillary or mandibular arch. Therefore, dental scan accuracy is improved as the scan substrate acts as a scan reference. Thus, the processing software can recognise and stitch together individual area scans with relative ease. Advantageously, this results in a scan of the maxillary or mandibular arch with increased accuracy. Beneficially, it also reduces the risk of misplacement or misalignment during dental procedures, and thus ultimately a reduced risk of patient discomfort, infection, gum recession, nerve, and tissue damage.

The invention will now be more particularly described, by way of example only, with reference to the accompanying drawings, in which:
Figure 1 shows a top-down view of a first embodiment of a dental scan aid in accordance with the first aspect of the invention;
Figure 2 shows a top-down view of the dental scan aid of Figure 1 in use, shown in place relative to a three-dimensional rendering of a patient's mouth;
Figure 3 shows a side perspective view of Figure 2; and
Figure 4 shows a schematic drawing of a second and third embodiment of a dental scan aid in accordance with the first aspect of the invention, both embodiments in use and attached to separate oral structures.

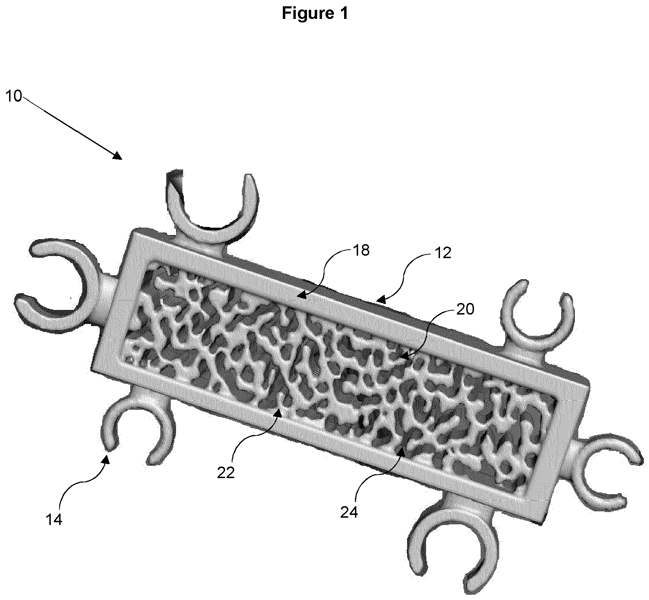

Referring to Figure 1, there is indicated a dental scan aid, referenced globally at 10. The dental scan aid 10 is particularly suitable for enhancing a scan accuracy of a maxillary or mandibular arch during a dental scan procedure.

The dental scan aid 10 comprises a scan substrate 12 and an oral attachment 14. The dental scan aid 10 is preferably formed as a unitary solid piece. The dental scan aid 10 is preferably made from a material that is durable and biocompatible. The scan substrate 12 is substantially planar.

The length of the scan substrate 12 is dimensioned to fit in the mouth of a patient. The scan substrate 12 preferably has a length of no more than 80 mm. The length of the scan substrate 12 is preferably between 2.5 mm and 70 mm. More preferably, the length of the scan substrate 12 is between 5 mm and 60 mm. The length of the scan substrate 12 is preferably complementary to the distance between two oral structures 16, or oral points of interest. The length of the scan substrate 12 is preferably shorter than a user's maxillary or mandibular arch. Therefore, it should be understood that the length of the scan substrate 12 may be made longer or shorter to accommodate different dental needs, procedures, and patients.

The width of the scan substrate 12 is preferably no more than 80 mm. The width of the scan substrate 12 is preferably between 2 and 50 mm. More preferably, the width of the scan substrate 12 is between 2.5 mm and 20 mm. Most preferably, the width of the scan is between 5 mm and 10 mm. As above, it is to be understood that the width of the scan substrate 12 may be made wider or narrower to accommodate different dental needs, procedures, and patients.

The ratio between the length and width of the scan substrate 12 is preferably 2:1, more preferably 4:1, and most preferably 3:1.

The depth of the scan substrate 12 is preferably no more than 25 mm. The depth of the scan substrate 12 is preferably between 2 mm and 10 mm. The depth of the scan substrate 12 is preferably complementary to the height of the oral structures 16.

The scan substrate 12 has a perimetric oral attachment support 18. The scan substrate 12 has a central portion 20 confined by the perimetric oral attachment support 18. The central portion 20 has an irregular morphology 22. The irregular morphology 22 may be described as randomised. The irregular morphology 22 in the depicted embodiment does not extend beyond the perimetric oral attachment support 18. However, it is envisaged that the irregular morphology 22 could comprise protrusions that do extend beyond the perimetric oral attachment support 18.

The irregular morphology 22 is defined by a plurality of void regions 24. The void regions 24 are irregularly shaped. Each irregularly shaped void region 24 has length, width, and depth. Preferably, less than 50% of the irregularly shaped void regions 24 have the same length and width. More preferably, less than 25% of the irregularly shaped void regions 24 have the same length and width. Most preferably, the length and width of each irregularly shaped void region 24 of the plurality of irregularly shaped void regions 24 is different or substantially different to another irregularly shaped void region 24.

In the depicted embodiment, the irregularly shaped void regions 24 extend through the depth of the scan substrate 12. However, it is envisaged that the irregularly shaped void regions 24 may be formed as surface indentations and thus have a depth less than the depth of the scan substrate 12. It is also envisaged that an irregularly shaped void region 24 of the plurality of irregularly shaped void regions 24 may have a different depth than a different irregularly shaped void region 24.

The central portion 20 preferably comprises 50% of the scan substrate 12. More preferably, the central portion 20 comprises 70% of the scan substrate 12. Even more preferably, the central portion 20 comprises 80% of the scan substrate 12. Most preferably, the central portion 20 comprises 90% of the scan substrate 12.

In the depicted embodiment, the dental scan aid 10 has a plurality of oral attachments 14. Two different types of oral attachments 14 are shown in Figure 1. One oral attachment 14 has a larger width than the other. Each longitudinal side has one oral attachment 14 of each type. The oral attachments 14 are arranged such that an oral attachment 14 with a larger width is opposite an oral attachment 14 with a narrower width. Each lateral end has a singular oral attachment 14. One lateral end has an oral attachment 14 with the larger width and the other lateral end has an oral attachment 14 with the narrower width.

The oral attachments 14 extend from the perimetric oral attachment support 18. The oral attachments 14 are shown extending from four faces of the perimetric oral attachment support 18. Preferably, the oral attachments 14 extend planar to the perimetric oral attachment support 18. The oral attachments 14 may be distributed around the perimetric oral attachment support 18 in any position. For example, one face of the perimetric oral attachment support 18 may have three oral attachments 14, whilst another face of the perimetric oral attachment support 18 may have none.

Each oral attachment 14 is preferably complementary in shape and dimension to an associated oral structure 16, that is, dimensioned to engage with the said oral structure 16. In this embodiment, the oral attachment 14 is made from a resiliently flexible material. The oral attachments 14 are 'C' shaped. The 'C' shape allows for push-fit engagement with an oral structure 16. The oral attachments 14 are depicted as being the same as one another. However, it is envisaged that the oral attachment 14 may be of varying types. For example, the oral attachment 14 may comprise a screw platform.

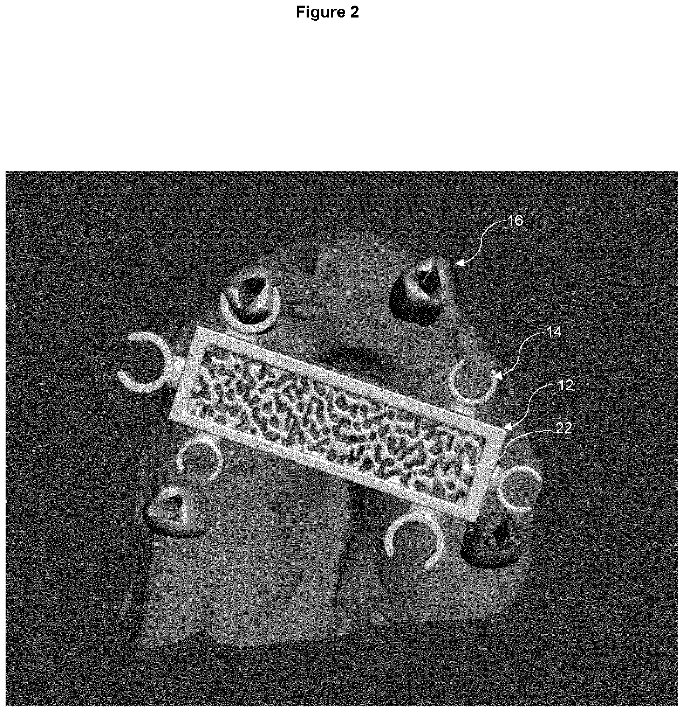

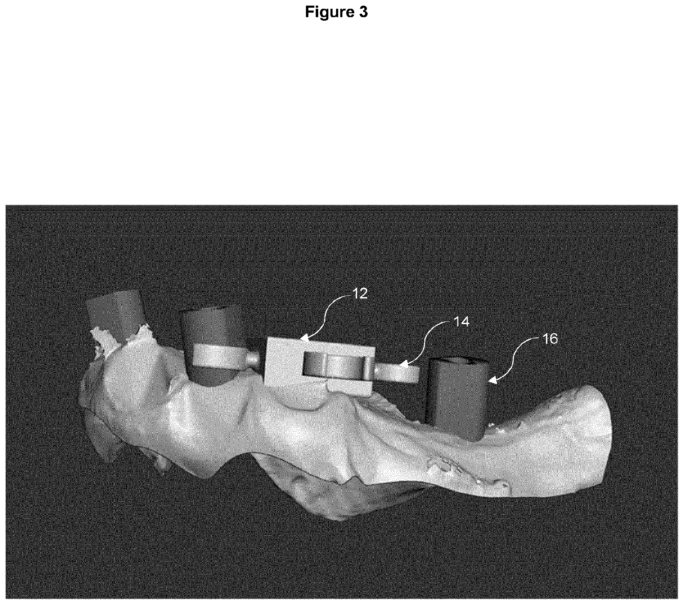

Figures 2 and 3 show the dental scan aid 10 in use. The dental scan aid 10 is attached to an oral structure 16. The oral structure 16 depicted is an intraoral scan body. However, possible oral structures 16 include and are not limited to: a tooth and/or an implant and/or a dental abutment. Indeed, where the patient is fully dentate, the teeth must be the oral structure 16 in question.

Firstly, a dental professional chooses a dental scan aid 10 of an appropriate length, width, and depth for its intended use. For example, for large edentulous areas, a longer and/or wider dental scan aid 10 may be preferred. Conversely, the length, width, and depth of the dental scan aid 10 may be dependent on the length and width of a patient's maxillary or mandibular arch.

The dental professional may also choose a dental scan aid 10 having a central portion 20 that encompasses a certain preferred percentage of the scan substrate 12. The central portion 20 may be defined as central within the scan substrate 12. Alternatively, the central portion 20 may be defined as central relative to the oral attachments 14. In this embodiment, the central portion 20 may not be central within the scan substrate 12. The central portion 20 may be recessed relative to an edge. The central portion 20 may be central relative to a defined singular axis. The depth of the irregularly shaped void regions 24 may also dictate a dental professional's choice.

The choice of dental scan aid 10 may also be driven by the type and/or number of oral attachments 14 provided on a dental scan aid 10. A dental professional may choose a dental scan aid 10 having oral attachments 14 that are all identical. Alternatively, a dental professional may choose a dental scan aid 10 having at least one different oral attachment 14.

A dental professional attaches the dental scan aid 10 to the oral structure 16 via an oral attachment 14. The oral attachment 14 depicted is attached to the oral structure 16 via push-fit engagement. The dental professional may then position the dental scan aid 10 as desired. This positioning may entail attaching the dental scan aid 10 to another oral structure 16 via a separate oral attachment 14. Conversely, the positioning may entail aligning the dental scan aid 10 over an edentulous area. Alignment over an edentulous area allows for increased scan accuracy.

A dental professional can then perform an intraoral scan of the of the maxillary and/or mandibular arch using an intraoral scanner. Once the dental scan is completed, the individual scan images taken by the intraoral scanning will be stitched together to give an overall scan of the maxillary and/or mandibular arch. The use of the dental scan aid 10 improves the accuracy of the overall can as it improves the ease and accuracy with the individual scan images are stitched together. The dental scan aid 10 acts as a background standard. The dental professional may use a scan program, implemented by a processor, that can recognise the irregular morphology 22 of the scan substrate 12, and thus determine how the individual scan images should be stitched together.

To remove the dental scan aids 10 from the resultant scan, the dental professional may perform a second scan of the maxillary and/or mandibular arch with only the oral structures 16 in place and the scanner may combine this information with the first scan. Alternatively, a dental professional may use a scan of the maxillary and/or mandibular arch without the dental scans and combine the scans. In both cases, the scan use uses positional information provided by the scan substrate 12. The position information is relative to the maxillary or mandibular arch.

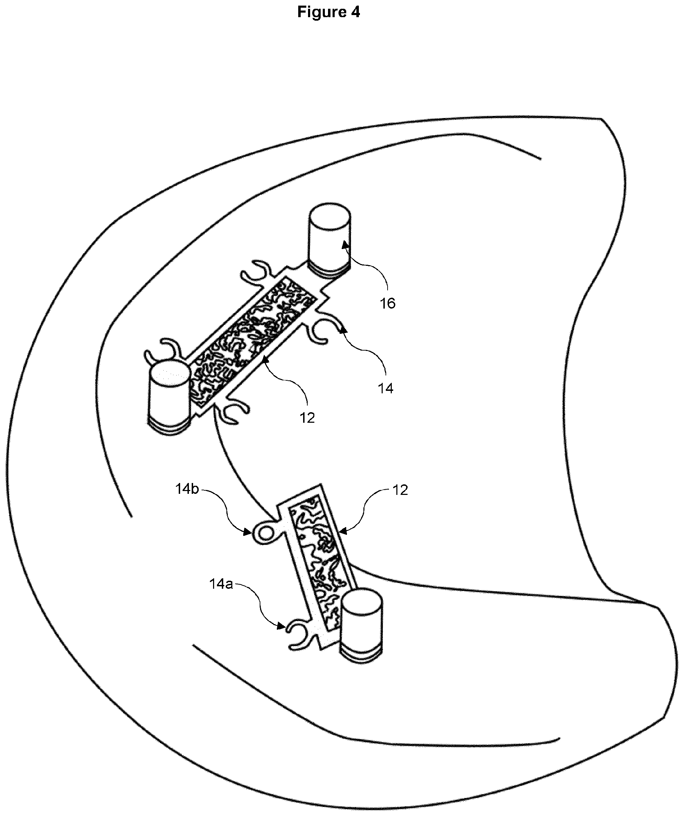

As shown in Figure 4, a plurality of dental scan aids 10 may be provided. At least one of the plurality of dental scan aids 10 may have a scan substrate 12 different to at least one other dental scan aid 10. The scan substrate 12 may differ in irregular morphology 22. For example, the irregular morphology 22 may be different and/or the area of the scan substrate 12 covered by the irregular morphology 22 may be different. Different irregular morphologies allow for differentiation between the dental scan aids 10 during a dental scan.

Alternatively, or additionally, the scan substrate 12 may be of a different length and/or width, when compared to the length and/or width of at least one other dental scan aid 10. The length and/or width of a patient's jaw and/or oral structures 16 may dictate the appropriate scan substrate 12 length and width.

Furthermore, as shown in Figure 4, the at least one dental scan aid 10 may have an oral attachment 14 different to an oral attachment 14 of at least one other dental scan aid 10. Furthermore, a dental scan aid 10 may have two unique styles of oral attachment 14. A push-fit engagement oral attachment 14a and a screw platform oral attachment 14b are shown. The screw platform oral attachment 14b may have a bore therethrough such that a screw may be inserted through the bore, and aligned with an oral structure 16 and the dental scan aid 10 screwed to the oral structure 16. Once in place, the screw preferably would sit flush with the screw platform oral attachment 14b.

The nature of the oral structure 16 that is fixed relative to the maxillary or mandibular arch may determine the dental scan aid 10 suitable. For example, for some dental structures, such as dental abutments, it may be preferable to use an oral attachment 14 that attaches to the oral structure 16 via push-fit engagement. In other circumstances it may be preferable to be able to screw the dental scan aid 10 to the oral structure 16, for example, when fixing to a bone-fixation screw, and thus having an oral attachment 14 comprising a screw platform may be useful. Additionally, the size of the oral structures 16 may dictate the type of oral attachment 14 that is preferred. It would be preferable for the size and dimension of the oral attachment 14 to be complementary to the size and dimension of the oral structure 16. A complementary fit may increase the efficiency and accuracy of the dental scan aid 10. Although not depicted or described, it is envisaged that other attachment means that allow for attachment of the dental scan aid to an oral structure would be suitable for use. It will be appreciated that a specific tooth bonding attachment will be required for engagement with teeth, for example, a click-fit engagement. This is necessary for fully dentate patients. It may be possible to provide selectably interchangeable oral attachments 14 for the dental scan aid 10, so that the oral attachments can be swapped for different patients. With tooth clasps or similar, these may be bespoke to a patient's teeth, and could indeed be 3D printed in situ as required.

Furthermore, the proposed dental procedure may dictate the form of the dental scan aid 10 which is suitable. It may be preferable to have different dental scan aids 10 attached to similar and/or different oral structures 16. It may also be preferable to have multiple dental scan aids 10 of varying widths and/or lengths attached to oral structures 16 of the mouth.

Once a dental professional has chosen their selected plurality of dental scan aids 10, the dental professional attaches each dental scan aid 10 of the plurality of dental scan aids 10 to a corresponding oral structure 16 in a patient's mouth.

The dental professional may attach one dental scan aid 10 to multiple oral structures 16. For example, it may be that one dental scan aid 10 is attached to two similar type oral structures 16, such as two dental abutments. In another example, it may be that the one dental scan aid 10 is attached to two distinct types of oral structure 16, such as an intraoral scan body and an implant. The dental professional may attach more than one dental scan aid 10 to a singular oral structure 16. The dental scan aids 10 may be arranged in a particular configuration that is determined by the desired dental procedure. The number of dental scan aids 10 used may vary per dental procedure and patient. The positioning of dental scan aids 10 may also vary per patient, even for similar dental procedures.

Once the dental scan aids 10 are attached, the dental professional may then perform a dental scan of the maxillary and/or mandibular arch. The dental scan will create individual scan images, which can then be processed together to show an overall or composite scan image of the maxillary and/or mandibular arch. It will be apparent, however, that advances in scanning technology may be able to direct image the maxillary and/or mandibular arch without any need for intermediate image reconstruction.

After the first scan of the maxillary and/or mandibular arch with the dental scan aids 10 in place, a dental professional may remove the dental scan aids 10, and then perform a second of the maxillary and/or mandibular arch with only the oral structures 16 in place. This second scan may be combined with the first scan to produce a scan that uses the positional data provided by the first scan, but without the dental scan aids 10 being optically present.

After scanning the maxillary and/or mandibular arch with the dental scan aids 10 in place, a dental professional may utilise a scan of the maxillary and/or mandibular arch without the dental scan aids 10 in place and overlay the two scans to provide a scan that utilises the positional data collected from using the dental scan aids 10 but removes them visually.

Without the dental scan aids 10, this scan image would be less accurate, as featureless areas, such as those awaiting dental reconstruction, reduce scan accuracy. Scan stitching of the individual scan images is less accurate.

Although not depicted, the design of the irregular morphology of the scan substrate may be made as follows. An initial shape may be designed, this shape may then be mirrored and rotated in different directions to create a design with irregular shapes. The mirroring and rotating process may be done randomly, so as to generate a randomised irregular design. This design may then be extended into three dimensions to result in a scannable three dimensional object with an irregular morphology.

For instance, the irregular morphology could be generated via three-dimensional extrusion of a two-dimensional surface which contains a randomly generated structure. Such a randomly generated surface could be generated by programming a noise function or random event multipliers that provide increased peaks and troughs in the surface, improving the irregularity of the morphology such that no two topographical areas are identical.

The peaks and troughs could be swapped with randomised holes, for instance, or shapes or alternative randomly generated additions to the surface, either alone or in combination, so that no two topographical areas are identical.

Additionally, although not depicted, it is envisaged that one oral attachment would be sufficient. Additionally, any appropriate number of oral attachments is envisaged as suitable.

Although the dental scan aid is described as being formed as a unitary solid piece, it is envisaged that the oral attachments may be detachable from the scan substrate. Indeed, it may be possible to form a modular dental scan aid which can be dimensioned to a correct size.

The present invention provides a dental scan aid for enhancing a scan accuracy of a maxillary or mandibular arch during a dental scan procedure. The dental scan aid has a scan substrate having at least in part an irregular morphology. The irregular morphology provides positional information relative to a maxillary or mandibular arch. The irregular morphology allows the scan substrate, and subsequently the dental scan aid to function as a referencing object. This increases the ease and accuracy with which processing software can stitch together scans generated by an intraoral scanner. Beneficially, this results in an overall more accurate scan of the maxillary or mandibular arch and any oral structures present. Therefore, the risk of patient discomfort, infection, gum recession, nerve, and tissue damage, due to inaccurate scan data, and thus an inaccurate dental procedure is reduced.

The words 'comprises/comprising' and the words 'having/including' when used herein with reference to the present invention are used to specify the presence of stated features, integers, steps, or components, but do not preclude the presence or addition of one or more other features, integers, steps, components, or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

The embodiments described above are provided by way of examples only, and various other modifications will be apparent to persons skilled in the field without departing from the scope of the invention as defined in the appended claims.

## Claims

1. A dental scan aid (10) for enhancing a scan accuracy of a maxillary or mandibular arch during a dental scan procedure, the dental scan aid (10) comprising:
a scan substrate (12);
an oral attachment (14) extending from the scan substrate (12), the oral attachment (14) being attachable to an oral structure (16) fixed relative to the said maxillary or mandibular arch, wherein the oral attachment (14) is configured to be attached to an oral structure (16) comprising any one of: an intraoral scan body and/or a tooth and/or an implant and/or a dental abutment; and
wherein
the scan substrate (12) is substantially planar and at least in part has a randomised irregular morphology (22) defined by a plurality of irregularly shaped void regions (24), the randomised irregular morphology (22) thereby providing positional information relative to the maxillary or mandibular arch to improve dental scan accuracy when the oral attachment (14) is attached to the said oral structure (16) during a dental scan procedure.

2. A dental scan aid (10) as claimed in claim 1, wherein the oral attachment (14) is resiliently flexible to allow for push-fit attachment.

3. A dental scan aid (10) as claimed in claim 1 or claim 2, wherein the oral attachment (14) comprises a screw platform to allow for dental scan aid (10) to be screwed to an oral structure (16).

4. A dental scan aid (10) as claimed in any one of the preceding claims, wherein the scan substrate (12) has a perimetric oral attachment (14) support to which the oral attachment (14) is connected, wherein the scan substrate (12) has a central portion (20) confined by the perimetric oral attachment support (18), and the central portion (20) has the said irregular morphology (22).

5. A dental scan aid (10) as claimed in any one of the preceding claims, wherein the dental scan aid (10) comprises a plurality of said oral attachments extending from the scan substrate (12).

6. A dental scan aid (10) as claimed in any one of the preceding claims, wherein the scan substrate (12) has a length of no greater than 80 mm.

7. A dental scan aid as claimed in any one of the preceding claims, wherein the irregularly shaped void regions (24) extend through a depth of the scan substrate (12).

8. A dental scan aid system comprising:
a plurality of dental scan aids (10) as claimed in any one of claims 1 to 7; and
wherein at least one of the plurality of dental scan aids (10) has a different irregular morphology (22) to at least one other dental scan aid (10) of the plurality of dental scan aids (10).

9. A dental scan aid system as claimed in claim 8, wherein at least one of the plurality of dental scan aids (10) has an oral attachment (14) different to at least one other dental scan aid (10).

10. A dental scan aid system as claimed in claim 8 or claim 9, wherein at least one of the plurality of dental scan aids (10) has a length and/or width different to at least one other dental scan aid (10).

11. A dental scan aid apparatus comprising:
a dental scan aid (10) as claimed in any one of claims 1 to 7; an intra oral scanner; and
and a processor for processing scan data collected by said intra oral scanner.

## Patentansprüche

1. Zahnscanhilfe (10) zum Steigern einer Scangenauigkeit eines Oberkieferbogens oder eines Unterkieferbogens während eines Zahnscanvorgangs, wobei die Zahnscanhilfe (10) umfasst:
ein Scansubstrat (12);
ein orales Anbringungsteil (14), das sich von dem Scansubstrat (12) erstreckt, wobei das orale Anbringungsteil (14) an einer oralen Struktur (16) anbringbar ist, die in Bezug auf den besagten Oberkieferbogen oder den besagten Unterkieferbogen fixiert ist, wobei das orale Anbringungsteil (14) dazu konfiguriert ist, an einer oralen Struktur (16) angebracht zu werden, umfassend einen beliebigen bzw. ein beliebiges von: einem Intraoralscankörper und/oder einem Zahn und/oder einem Implantat und/oder einem Zahn-Abutment; und
wobei das Scansubstrat (12) im Wesentlichen planar ist und zumindest zum Teil eine randomisierte unregelmäßige Morphologie (22) aufweist, die durch eine Vielzahl von unregelmäßig geformten Leerraumregionen (24) definiert wird, wobei die randomisierte unregelmäßige Morphologie (22) dadurch Positionsinformationen in Bezug auf den Oberkiefer- oder den Unterkieferbogen bereitstellt, um eine Zahnscangenauigkeit zu verbessern, wenn das orale Anbringungsteil (14) während eines Zahnscanvorgangs an der besagten oralen Struktur (16) angebracht ist.

2. Zahnscanhilfe (10) nach Anspruch 1, wobei das orale Anbringungsteil (14) elastisch flexibel ist, um eine Steckanbringung zu ermöglichen.

3. Zahnscanhilfe (10) nach Anspruch 1 oder Anspruch 2, wobei das orale Anbringungsteil (14) eine Schraubenplattform umfasst, um ein Schrauben der Zahnscanhilfe (10) an eine orale Struktur (16) zu ermöglichen.

4. Zahnscanhilfe (10) nach einem der vorhergehenden Ansprüche, wobei das Scansubstrat (12) eine umlaufende Stütze für das orale Anbringungsteil (14) aufweist, mit der das orale Anbringungsteil (14) verbunden wird, wobei das Scansubstrat (12) einen zentralen Abschnitt (20) aufweist, der durch die umlaufende Stütze (18) für das orale Anbringungsteil begrenzt wird, und der zentrale Abschnitt (20) die besagte unregelmäßige Morphologie (22) aufweist.

5. Zahnscanhilfe (10) nach einem der vorhergehenden Ansprüche, wobei die Zahnscanhilfe (10) eine Vielzahl der besagten oralen Anbringungsteile umfasst, die sich von dem Scansubstrat (12) erstrecken.

6. Zahnscanhilfe (10) nach einem der vorhergehenden Ansprüche, wobei das Scansubstrat (12) eine Länge von nicht mehr als 80 mm aufweist.

7. Zahnscanhilfe nach einem der vorhergehenden Ansprüche, wobei sich die unregelmäßig geformten Leerraumregionen (24) durch eine Tiefe des Scansubstrats (12) erstrecken.

8. Zahnscanhilfssystem, umfassend:
eine Vielzahl von Zahnscanhilfen (10) nach einem der Ansprüche 1 bis 7; und
wobei mindestens eine der Vielzahl von Zahnscanhilfen (10) eine unterschiedliche unregelmäßige Morphologie (22) zu mindestens einer anderen Zahnscanhilfe (10) der Vielzahl von Zahnscanhilfen (10) aufweist.

9. Zahnscanhilfssystem nach Anspruch 8, wobei mindestens eine der Vielzahl von Zahnscanhilfen (10) ein orales Anbringungsteil (14) aufweist, das sich von mindestens einer anderen Zahnscanhilfe (10) unterscheidet.

10. Zahnscanhilfssystem nach Anspruch 8 oder Anspruch 9, wobei mindestens eine der Vielzahl von Zahnscanhilfen (10) eine Länge und/oder eine Breite aufweist, die sich von mindestens einer anderen Zahnscanhilfe (10) unterscheiden.

11. Zahnscanhilfsvorrichtung, umfassend:
eine Zahnscanhilfe (10) nach einem der Ansprüche 1 bis 7;
einen Intraoralscanner; und
und einen Prozessor zum Verarbeiten von Scandaten, die von dem besagten Intraoralscanner gesammelt werden.

## Revendications

1. Une aide à la numérisation dentaire (10) destinée à améliorer la précision de numérisation d'une arcade maxillaire ou mandibulaire lors d'une procédure de numérisation dentaire, ladite aide à la numérisation dentaire (10) comprenant :
un substrat de numérisation (12) ;
un élément de fixation buccale (14) s'étendant à partir du substrat de numérisation (12), ledit élément de fixation buccale (14) étant apte à être fixé à une structure buccale (16) solidaire de ladite arcade maxillaire ou mandibulaire, dans lequel ledit élément de fixation buccale (14) est configuré pour être fixé à une structure buccale (16) comprenant l'un quelconque des éléments suivants : un corps de numérisation intraoral et/ou une dent et/ou un implant et/ou un pilier dentaire ; et
dans lequel le substrat de numérisation (12) est sensiblement plan et présente, au moins en partie, une morphologie irrégulière aléatoire (22) définie par une pluralité de régions vides de forme irrégulière (24), ladite morphologie irrégulière aléatoire (22) fournissant ainsi des informations de positionnement par rapport à l'arcade maxillaire ou mandibulaire afin d'améliorer la précision de la numérisation dentaire lorsque l'élément de fixation buccale (14) est fixé à ladite structure buccale (16) au cours d'une procédure de numérisation dentaire.

2. Une aide à la numérisation dentaire (10) selon la revendication 1, dans laquelle l'élément de fixation buccale (14) est élastiquement flexible de manière à permettre une fixation par emboîtement.

3. Une aide à la numérisation dentaire (10) selon la revendication 1 ou la revendication 2, dans laquelle l'élément de fixation buccale (14) comprend une plate-forme filetée permettant de visser l'aide à la numérisation dentaire (10) sur une structure buccale (16).

4. Une aide à la numérisation dentaire (10) selon l'une quelconque des revendications précédentes, dans laquelle le substrat de numérisation (12) comporte un support d'élément de fixation buccale périmétrique (14) auquel l'élément de fixation buccale (14) est connecté, et dans laquelle le substrat de numérisation (12) comporte une partie centrale (20) confinée par l'élément de fixation buccale périmétrique (18), et la partie centrale (20) présente ladite morphologie irrégulière (22).

5. Une aide à la numérisation dentaire (10) selon l'une quelconque des revendications précédentes, dans laquelle l'aide à la numérisation dentaire (10) comprend une pluralité desdits éléments de fixation buccale (14) s'étendant à partir du substrat de numérisation (12).

6. Une aide à la numérisation dentaire (10) selon l'une quelconque des revendications précédentes, dans laquelle le substrat de numérisation (12) présente une longueur ne dépassant pas 80 mm.

7. Un aide à la numérisation dentaire selon l'une quelconque des revendications précédentes, dans laquelle les régions vides de forme irrégulière (24) s'étendent sur toute la profondeur du substrat de numérisation (12).

8. Un système d'aide à la numérisation dentaire comprenant :
une pluralité d'aides à la numérisation dentaire (10) selon l'une quelconque des revendications 1 à 7 ; et
dans laquelle au moins une de la pluralité des aides à la numérisation dentaire (10) présente une morphologie irrégulière (22) différente d'au moins une autre aide à la numérisation dentaire (10) de la pluralité d'aides à la numérisation dentaire (10).

9. Un système d'aide à la numérisation dentaire selon la revendication 8, dans lequel au moins l'une des aides à la numérisation dentaire (10) de ladite pluralité présente un élément de fixation buccale (14) différent d'au moins une autre aide à la numérisation dentaire (10).

10. Un système d'aide à la numérisation dentaire selon la revendication 8 ou la revendication 9, dans lequel au moins l'une des aides à la numérisation dentaire (10) de ladite pluralité présente une longueur et/ou une largeur différente d'au moins une autre aide à la numérisation dentaire (10).

11. Une aide à la numérisation dentaire comprenant :
une aide à la numérisation dentaire (10) selon l'une quelconque des revendications 1 à 7 ;
un scanner intra-oral ; et
un processeur destiné au traitement des données de numérisation collectées par ledit scanner intraoral.
